# EUROPEAN PATENT SPECIFICATION

(11) **EP 1 237 620 B1**
(45) Date of publication and mention of the grant of the patent: **24.11.2004**
(21) Application number: 99966321.4
(22) Date of filing: 15.12.1999
(51) Int. Cl.: A61N 1/32

(54) **ELECTROPORATION DEVICE**
VORRICHTUNG ZUR ELEKTROPORATION
DISPOSITIF D'ELECTROPORATION

(43) Date of publication of application: 11.09.2002
(73) Proprietor: University of South Florida, Tampa, Florida 33620 (US)
(72) Inventor: HELLER, Richard, Temple Terrace, FL 33617 (US); GILBERT, Richard, Tampa, FL 33617 (US); JAROSZESKI, Mark, J., Tampa, FL 33613 (US)
(74) Representative: Tomkinson, Alex
(86) International application number: PCT/US1999/029911
(87) International publication number: WO 2001/043817

(56) References cited:
- WO-A-94/22526
- WO-A-95/23211
- WO-A-96/00111
- WO-A-96/39226
- WO-A-98/47562
- WO-A-99/37358

## Description

### BACKGROUND OF THE INVENTION

### Field of the Invention

The present invention relates to apparatus for delivering molecules into a target cell, and, more particularly, to such methods and apparatus for achieving such delivery through electroporation and electromigration.

### Description of Related Art

The effect of electromagnetic fields on cell membranes has been studied since the 1960s. Early research focused on describing observations that an applied electric field can reversibly break down cell membranes *in vitro*. Throughout the 1970s the topic was more common in the literature and continued to focus on describing the phenomenon that resulted from brief exposure to intense electric fields as well as the entry of exogenous molecules to the cell interior as a result of membrane breakdown. Applications began to emerge along with a better understanding of reversible membrane breakdown in the 1980s.

Prior research led to the current understanding that exposure of cells to intense electric fields for brief periods of time temporarily destabilized membranes. This effect has been described as a dielectric breakdown due to an induced transmembrane potential, and was termed "electroporation," or "electropermeabilization," because it was observed that molecules that do not normally pass through the membrane gain intracellular access after the cells were treated with electric fields. The porated state was noted to be temporary. Typically, cells remain in a destabilized state on the order of minutes after electrical treatment ceases.

The physical nature of electroporation makes it universally applicable. A variety of procedures utilize this type of treatment, which gives temporary access to the cytosol. These include production on monoclonal antibodies, cell-cell fusion, cell-tissue fusion, insertion of membrane proteins, and genetic transformation. In addition, dyes and fluorescent molecules have been used to investigate the phenomenon of electroporation. A notable example of loading molecules into cells *in vivo* is electrochemotherapy. The procedure utilizes a drug combined with electric pulses as a means for loading tumor cells with an anticancer drug, and has been performed in a number of animal models and in clinical trials by the present inventors. Also, plasmid DNA has been loaded into rat liver cells *in vivo* (Heller et al., *FEBS Lett*. **389,** 225-28).

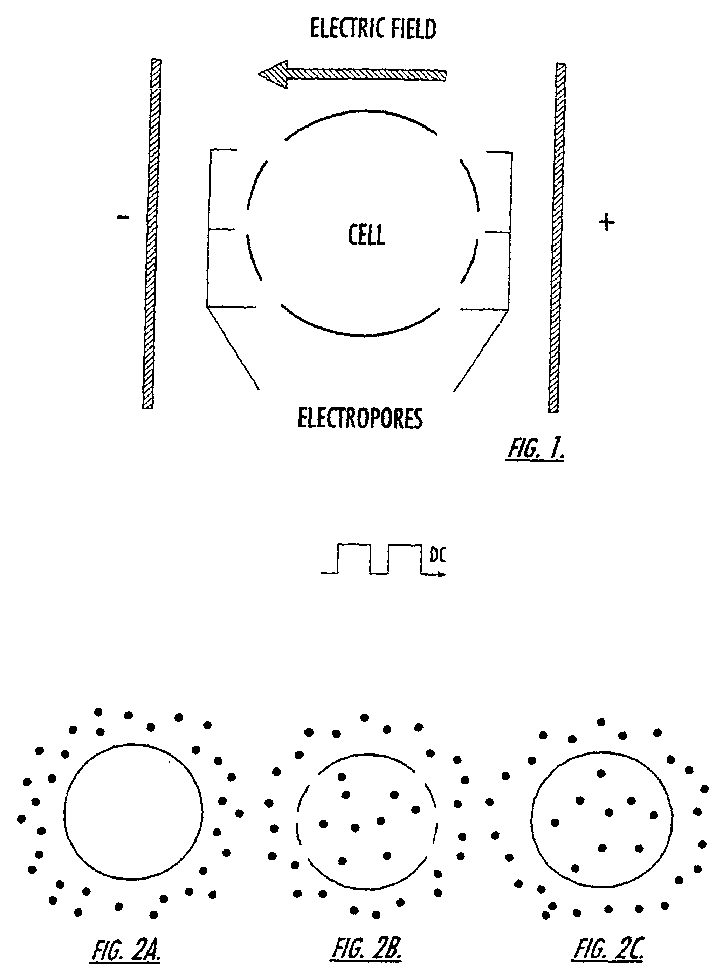

Protocols for the use of electroporation to load cells *in vitro* typically use a suspension of single cells or cells that are attached in a planar manner to a growth surface. *In vivo* electroporation is more complex because tissues are involved. Tissues are composed of individual cells that collectively make up a three-dimensional structure. In either case, the effects on the cell are the same. FIGURE 1 illustrates details of the electrical treatment procedure. Electrodes and electrode arrays for delivering electrical waveforms for therapeutic benefit, including inducing electroporation, have been described by Bernard (WO 98/47562).

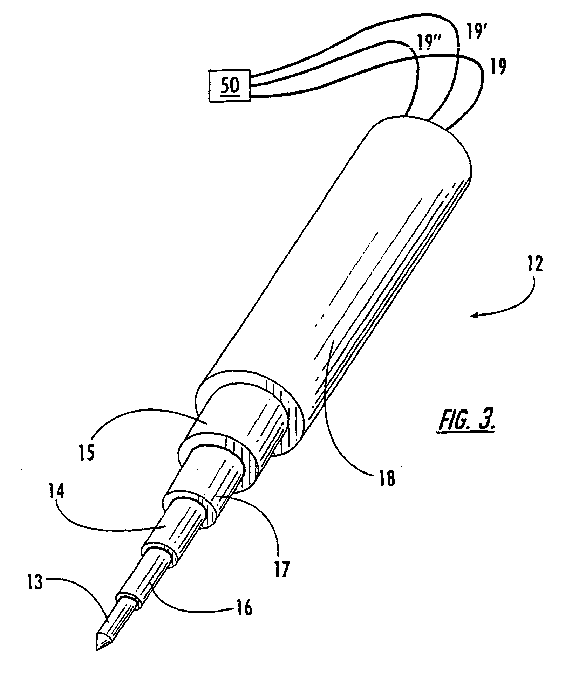

The loading of molecules by electroporation *in vitro* as well as *in vivo* is typically carried out by first exposing the cells or tissue of interest to a drug or other molecule (FIG. 2). The cells or tissue are then exposed to electric fields by administering one or more direct current pulses. Electrical treatment is conducted in a manner that results in a temporary membrane destabilization with minimal cytotoxicity. The intensity of electrical treatment is typically described by the magnitude of the applied electric field. This field is defined as the voltage applied to the electrodes divided by the distance between the electrodes. Electric field strengths ranging from 1000 to 5000 V/cm have been used for delivering molecules *in vivo* and are also specific to the cells or tissue under investigation. Pulses are usually rectangular in shape; however, exponentially decaying pulses have also been used. The duration of each pulse is called *pulse width.* Molecule loading has been performed with pulse widths ranging from microseconds (µs) to milliseconds (ms). The number of pulses delivered has ranged from one to eight. Typically, multiple pulses are utilized during electrical treatment.

For molecules to be delivered to the cell interior by electroporation, it is important that the molecule of interest be near the exterior of the cell membrane when in the cell is in a permeabilized state. It is also important to have molecules near substantially all cells within a treated tissue volume in order to provide efficient delivery to substantially all cells within the treatment volume.

Currently, molecules are injected systemically, via methods well know to those of skill in the art, or directly into the treatment site. No attempt is made to produce a specific distribution of molecules is sufficient to provide effective delivery to substantially all the cells.

Electropermeabilization of tumor cell membranes in vivo has been reported (Rols et al., *Nature Biotechnology* **16,** 173, 1998) using applied electric pulses from surface electrodes in contact with the skin. A protein can be transferred into or and expressed by the cells by incorporating either the protein or a plasmid carrying a reporter gene. The efficiencies of transfer for the protein and plasmid were, respectively, 20 and 4%.

A first type of electrode known in the art comprises parallel-plate electrodes placed on opposite sides of the tumor. Other electrodes known in the art at the present time comprise needles that are inserted into or around the tissue of interest. One such example is disclosed in WO96/39926. A further type of electrode arrangement is disclosed in US538069 which includes two axially spaced apart electrodes on a penetrator which is inserted into a target tissue. However, these prior art arrangements teach to the electric fields being applied in only two dimensions of the three-dimensional tissue matrix. This limits the area of each cell that can be electroporated (FIG.1), which reduces delivery efficiency.

A two-dimensional array of needles has also been disclosed (Gilbert et al., *Biochim. Biophys. Acta* **1334,** 9, 1997; U.S. Pat. No. 5,702,359) in which circularly disposed pairs of needles surround a target tissue. Pulses of opposite polarity are applied across each pair of needles in a predetermined sequence, which has been shown to improve tumor regression in a mouse melanoma study.

Electrodes and methods known in the art do not provide molecule movement during the preelectroporation period to enhance molecular distribution nor in the postelectroporation time period, when the cells are in a state of increased membrane permeability. The movement of molecules within the tissue is believed to effect an increase in the delivered quantity of molecules by enhancing movement into the cells.

WO98/47562 discloses means for allowing movement of the molecule during the pre-electroporation period or post-electroporation period comprising a support and at least first and second members affixed to and extending away from the support. The electrode arrangement includes three needle electrodes which form a triangle in the plane intersecting the electrodes. The document states that two dimensions are established by the geometry of the three electrode array and the third dimension is established by the length of the conductive region of the electrodes. However, the electrical field cannot be varied along the length of the third dimension, thereby still limiting the area of the target tissue that can be electroporated and provides no flexibility as to the strength of the field in the third dimension to allow differences in electroporation or molecule movement in the pre-electroporation period or post-electroporation period along the third dimension.

### SUMMARY OF THE INVENTION

It is therefore and object of the present invention to provide an improved device for manipulating molecules adjacent and/or within a target tissue site.

It is an additional object to provide such a device for manipulating molecules in three-dimensional space such as a tissue volume element.

It is a further object to provide such a device that can provide a desired electromagnetic field distribution adjacent and/or within a target tissue.

It is another object to provide such a device that can be configured to activate a multicomponent labile system at a desired site.

It is yet an additional object to provide a system for effecting tumor reduction.

It is yet a further object to provide a system for effecting in vivo gene transfer to cells via electroporation.

These objects and others are attained by the present invention, a device for manipulating a molecule in vivo relative to a target tissue in three dimensions. The device comprises a support, and at least first and second members affixed to and extending away from the support. The first member includes at least one discrete electrode and the second member includes at least two discrete electrodes, each electrode in independent circuit communication with a respective portion of a source of electrical energy and therefore being differentially activable. The electrodes are disposed in axially spaced-apart relation along the member.

The discrete electrodes are configured to establish a first electromagnetic field in vivo between selected electrodes sufficient to manipulate a molecule translationally relative to a target tissue. The electrodes are further configured to establish a second electromagnetic field sufficient to cause transient permeability of a cell membrane within the target tissue.

Such an arrangement permits triangulation between the independently activatble electrodes.

In a particular embodiment the electrodes are also configured to establish a third electromagnetic field sufficient to manipulate a molecule translationally following the second filed to continue enhancing molecular distribution and/or uptake at cells. Typically the first and the third field levels will be lower than that of the second, although this is not intended as a limitation.

In a particular embodiment the electrodes are activatable in a predetermined sequence, which may include sequential or simultaneous activation of any or all of the electrodes.

The device can be used, for example, with alternating current, direct current, pulsed alternating current, pulsed direct current, high- and low-voltage alternating current with variable frequency and amplitude, variable direct current waveforms, variable alternating current signals biased with variable direct current_waveforms, and variable alternating current signals biased with constant direct current.

The device as described above may be used to enhance the delivery of a molecule such as a bioactive molecule, nucleic acid, amino acid, polypeptide, protein, antibody, glycoprotein, enzyme, oligonucleotide, plasmid DNA, chromosome, or drug, although this list is not intended to be exhaustive or limiting. The device may be used to cause the electromigration of at least two components of a multicomponent reactive system into apposition to permit a reaction to occur at a desired target tissue site. The target tissue may comprise a tumor, and organ, or wound site.

The features that characterize the invention, both as to organization and method of operation, together with further objects and advantages thereof, will be better understood from the following description used in conjunction with the accompanying drawing. It is to be expressly understood that the drawing is for the purpose of illustration and description and is not intended as a definition of the limits of the invention. These and other objects attained, and advantages offered, by the present invention will become more fully apparent as the description that now follows is read in conjunction with the accompanying drawing.

### BRIEF DESCRIPTION OF THE DRAWINGS

**FIG. 1** (prior art) Conceptual two-dimensional depiction of electroporation of a cell subjected to an electromagnetic field. Regions of membrane breakdown, depicted as pores, are formed at the ends of cells facing the electrodes. Electromagnetic field exposure is achieved by applying a potential between electrodes - and +.
**FIG. 2** (prior art) The process of delivering molecules by electroporation. FIG. 2A. Cells *in vitro* or *in vivo* are exposed to the molecule of interest. FIG. 2B. Direct current pulses are administered to the cells to cause a temporary membrane destabilization that allows the molecules to more freely enter the cell interior. FIG. 2C. Cells return to their normal state after pulsation, leaving the molecule within the cells.
**FIG. 3** A first embodiment of one electrode-bearing member of a molecule manipulator, including radially disposed coaxial electrodes spaced apart by nonconductive material.
**FIG. 4** A molecule manipulator including a support and plural members as in FIG. 3.
**FIG. 5** A second embodiment of one electrode-bearing member of a molecule manipulator, including circumferential bands of electrodes disposed along a generally cylindrical electrode.
**FIG. 6** A molecule manipulator including a support and plural members as in FIG. 5.
**FIG. 7** The use of a molecule manipulator to bring components of a multicomponent reactive system into apposition at a target tissue site.

### DETAILED DESCRIPTION OF THE PREFERRED EMBODIMENTS

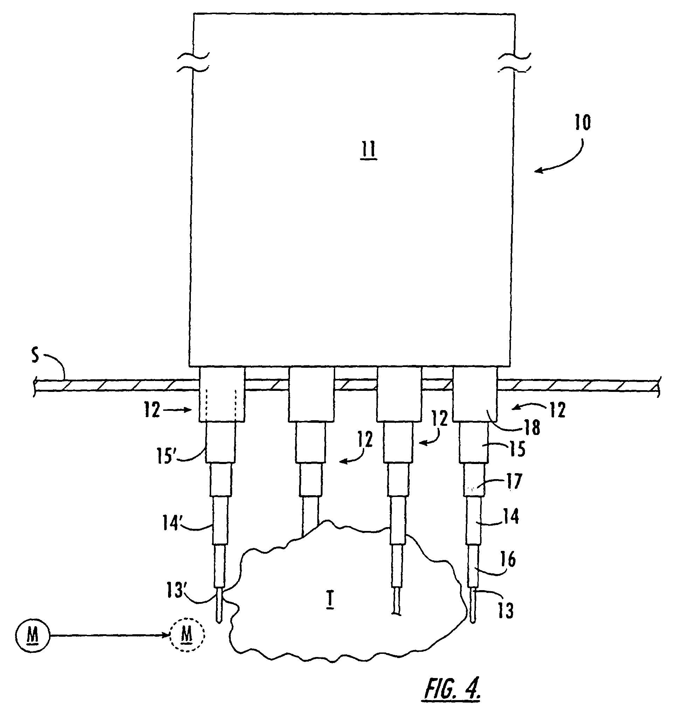

A description of the preferred embodiments of the present invention will now be presented with reference to FIGS. 3-7.

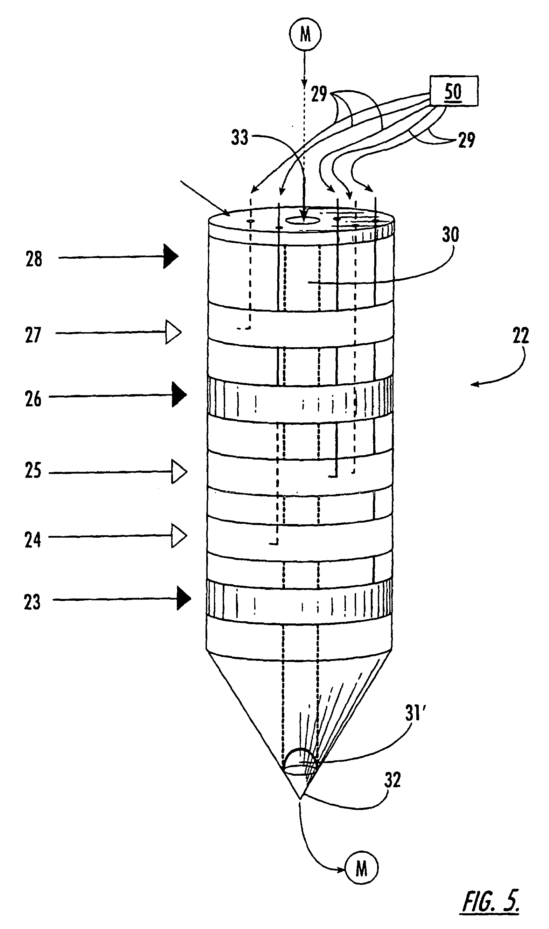

A first embodiment of a device **10** for manipulating a molecule **M** *in vivo* relative to a target tissue **T** (FIGS. 3 and 4) comprises a support **11** that is adapted to reside outside the site of the target tissue **T**, here shown as atop the skin **S**, although this is not intended as a limitation. At least one member **12**, here shown as four members **12**, are affixed to and extend away from the support **11**. The members **12** are preferably disposed about the support **11** in spaced relation from each other and are configured to surround a periphery of and/or penetrate into at least a portion of the target tissue **T,** with at least a lower portion of the members **12** penetrating the skin **S** (or other organ or tissue) to reach the target tissue **T**. Here the target tissue is **T** represented as a tumor, although this is not intended to be a limitation.

Each member **12** has at least two discrete electrodes disposed in axially spaced-apart relation from each other, here shown as three electrode portions **13,14,15.** Each electrode **13-15** is in circuit communication with a respective portion of a source **50** of electrical energy. The term "in circuit communication" as used herein is used to describe (1) devices that are directly or indirectly electrically connected with each other; (2) devices having other devices or combinations of devices (e.g., breakers, relays, buffers, drivers, transmitters, receivers, and decoders) between them; (3) devices in optical communication with each other (via, e.g., an optoisolator or fiber optic link); (4) devices in electromagnetic communication with each other (via, e.g., radio frequency transmitter and receiver pair); (5) devices connected by and through other structures allowing them to communicate with each other; and (6) any combination of any of the above.

In a preferred embodiment this source comprises a pulse generator such as is known in the art (e.g.,a PA-2000 or PA-4000, both from Cyto Pulse Sciences, Inc., Columbia, MD; a T820, BTX, Inc., San Diego, CA) and adapted to deliver pulses of a predetermined shape, voltage, duration, and separation. In particular, the source **50** should be adapted to deliver voltage to each electrode **13-15** for establishing a lower-level and a higher-level electromagnetic field *in vivo* between selected electrodes. Selective control of the application of electrical signals between the individual electrodes can be accomplished in different ways, e.g., via the PA-201 Programmable Pulse Switch in combination with the PA-4000 generator (both from Cyto Pulse Sciences, Inc., Columbia, MD) or it can be done manually, mechanically, or electrically.

The lower-level field is for manipulating (e.g., causing the electromigration of) the molecule **M** in three-dimensional fashion relative to the target tissue **T**, here shown as a mass. The higher-level field is for causing transient permeability of a cell membrane within the target tissue **T**. Such a permeability is useful for permitting the molecule **M** to enter the interior of the cell (see FIGS. 1 and 2). A lower-level field can also be applied following the application of a higher-level field to enhance molecule distribution within the target tissue and/or cause movement of the molecule(s) into the cell interior of the permeabilized cell by electroporation.

In the first embodiment, the member **12** comprises an elongated and pointed core electrode **13** that is made of a conductive material. A nonconductive insulator sleeve **16** is positioned in surrounding relation to a portion of the core electrode **13**, with a bottom portion of the core electrode **13** protruding and thus exposed.

A first outer electrode **14** is positioned in surrounding relation to a portion of the sleeve **16,** with a bottom portion of the sleeve **16** protruding therefrom and thus exposed.

A second sleeve **17** is positioned in surrounding relation to a portion of the first outer electrode **14**, with a bottom portion of the second electrode **14** protruding therefrom and thus exposed.

A second outer electrode **15** is positioned in surrounding relation to a portion of the second sleeve **17,** with a bottom portion of the second sleeve **17** protruding therefrom and thus exposed.

A third sleeve **18** is positioned in surrounding relation to a portion of the second outer electrode **15**, with a bottom portion of the second outer electrode **15** protruding therefrom and thus exposed.

In a prototype embodiment, which is not intended as a limitation, the device **10** comprises a stainless steel 30-gauge needle as the core electrode **13** placed in the hollow space of a 25-gauge hypodermic tubing as the first outer electrode **14** with a layer of nonconductive insulation between the electrodes **13,14** as the first sleeve **16.** A layer of insulation is placed over the first outer electrode **14** to serve as second sleeve **17,** and a section of 23-gauge tubing, serving as second outer electrode **15**, is placed over the second sleeve **17**. Insulation placed over the second outer electrode **15** serves as third sleeve **18.** The bottom portions of each of these elements are exposed to form a series of conductive bands **13-15** separated by nonconductive regions **16-18**.

This arrangement provides three electrodes exposed to the exterior with an insulator between each adjacent pair of electrodes. It can be appreciated by one of skill in the art that any number of electrodes and insulators could be successively configured in overlapping fashion to produce a multielectrode member tailored to a particular application based upon such considerations as, for example, the size of the target tissue and the space available.

Each electrode **13-15** has an independent lead **19,19', 19",** respectively, affixed to its top end to provide the circuit communication with the pulse generator.

In use the members **12** are typically arranged in opposing spaced-apart pairs, so that at least one electrode on each of a pair of members **12** can be adapted to provide at least one pair of opposite-polarity voltages approximately simultaneously. Further, it may be desired to selectively apply electrical potentials to each electrode pair in a predetermined pattern. Such a means for imposing a preselected pattern may include, for example, a software program for driving a pulse generator to deliver signals to each selected electrode in the preselected pattern.

It can be seen that three-dimensional manipulation can be effected by activating opposing pairs of electrodes at different axial levels to induce molecular movement and/or electropermeabilization along a desired pathway. For example, activating the pair **13-13'** would induce movement generally in a plane normal to the member **12,** whereas activating the pair **13-15'** would induce movement at an angle relative to the plane. These presupposed movements are, of course, subject to other conditions within and surrounding the tissue, and are only to be construed as relative and indicative of possible general directionalities achievable by the devices of the present invention.

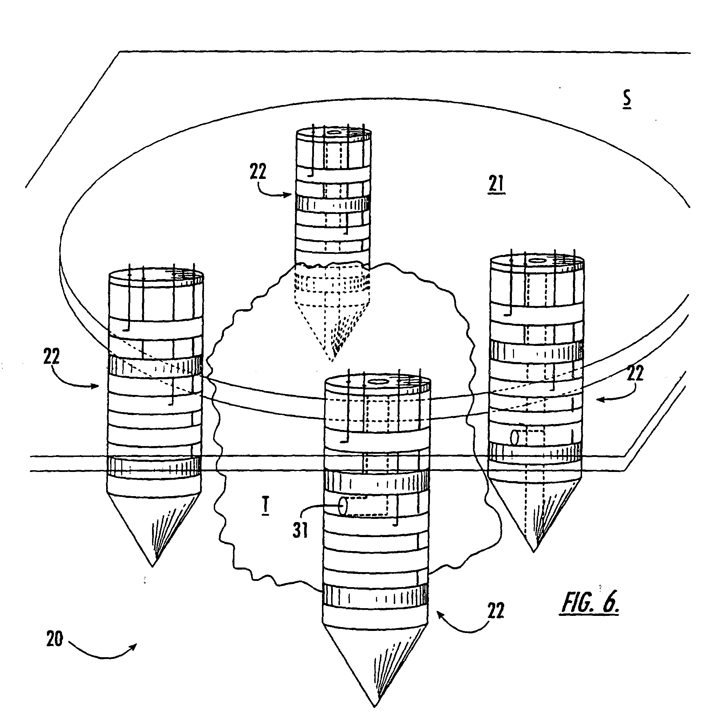

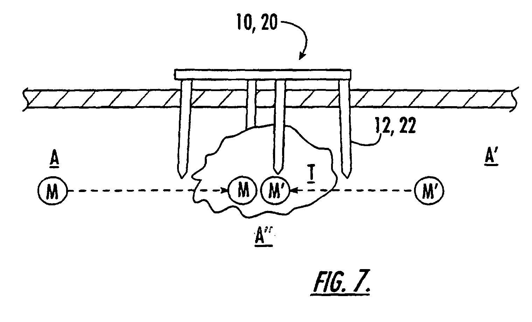

A second embodiment of a device **20** for manipulating a molecule **M** *in vivo* relative to a target tissue T (FIGS. 5 and 6) comprises a support **21** and at least one member 22, here shown as four members **22,** affixed to and extending away from the support **21.** The members **22** are preferably disposed about the support **21** in spaced relation from each other and are configured to surround a periphery of and/or penetrate into at least a portion of the target tissue **T**. In FIG. 6 the target tissue is **T** represented as a tumor, although this is not intended to be a limitation.

Each member **22** has at least two discrete electrodes, here shown as five electrodes **23-27** configured as circumferential rings disposed about a generally cylindrical nonconducting core post **28.** As above, each electrode **23-27** is in independent circuit communication with a respective portion of a source **50** of electrical energy. Such independent circuit communication may be formed, for example, by respective insulated leads **29** extending through the core **28** from the top end to each ring electrode **23-27**.

Additionally, the core **28** may have a lumen **30** therein extending from a top opening **33** for dispensing a molecule **M** to a tissue through a portal **31**. The portal **31** in one embodiment may be adjacent an electrode or may **31'** be at the core's tip **32,** which in a particular embodiment may be pointed.

Several methods of using the device of the present invention will now be disclosed. These methods will be illustrated with the device **10** described above, although this is not intended as a limitation, since either device **10** or **20** could be used therein, or other equivalents appreciated by one of skill in the art.

A first embodiment comprises a method for achieving an improved distribution and delivery of a desired molecule **M** into a target tissue **T.** This method comprises the steps of inserting at least one elongated member **12** into a body tissue generally adjacent and/or within a target tissue **T**. Such an insertion may be aided by any of the visualization techniques known in the art, such as computed tomography, ultrasound, x-radiography, although these are not intended as limitations. A substance that includes the desired molecule **M**, such as a solution thereof, is introduced into the body into an area near the target tissue **T**, either before or after the insertion of the device **10**. A particular introduction method comprises delivering the substance through a lumen **30** and portal **31** as in the embodiment of FIGS. 5 and 6. Any other method of introduction such as known to one of skill in thevart may also be employed.

A first electrical potential is established between a pair of electrodes **13-15'** that is sufficient to cause a redistribution and electromigration of the desired molecule **M** toward the target tissue T. Then subsequently a second electrical potential is established between a pair of electrodes, which may or may not be the same electrode pair as previously activated. The second potential has a sufficiently high electrical potential to cause electroporation in the target tissue T to enhancing a movement of the desired molecule **M** into a cell. An additional, third, low-level field after electroporation induction can be applied to further enhance molecular movement into cells and/or redistribution. Exemplary pulse magnitude and duration ranges include, but are not intended to be limited to, 1-10,000 volts/cm for a duration in the nanosecond to second range. A particular embodiment comprises a pulse or plurality of pulses in a range of 1-500 V/cm for a duration in the millisecond range for the first and the third potentials and a pulse or plurality of pulses in a range of 750-1500 V/cm in the microsecond range for the second potential. Naturally these values are not intended to be limiting, and one of skill in the art will appreciate that, for example, shorter pulses of higher magnitude or longer pulses of lower magnitude may be employed as required to achieve the desired effect.

A second method is for delivering a bioactive molecule to a subcutaneous target tissue **T**. This method comprises the steps of, as above, introducing a substance containing the bioactive molecule **M** systemically or into a subcutaneous area adjacent the target tissue **T.** A device such as device **10** is inserted into a body tissue generally adjacent a target tissue **T**, and electrode pairs are again activated at a low and high and again low level to achieve, respectively, an electromigration of the bioactive molecule **M** adjacent and within the target tissue **T,** an electroporation of a cell membrane within the target tissue **T** sufficient to permit entry of the bioactive molecule M into the cell interior, and additional electromigration within the target tissue.

A third method (FIG. 7) is for bringing two molecules **M,M'** into apposition at a desired target tissue site **T** for permitting a reaction therebetween, as in multicomponent reactive system, or a cell "bomb." This method comprises the steps of introducing a substance containing a first molecule **M** into a first area **A** adjacent and/or within the target tissue site T and introducing a substance containing a second molecule **M'** into a second area **A'** adjacent and/or within the target tissue site **T**.

Next an electromigration of the first **M** and the second molecule **M'** is caused to a third area **A"** that is adjacent and/or within the target tissue site **T**. The third area A" may actually comprise the first **A** or the second area **A'**, or another area distinct therefrom.

Next the first **M** and the second **M'** molecule are permitted to react at the third area **A"**.

It may be appreciated by one skilled in the art that additional embodiments may be contemplated, including different electrode configurations adapted to provide high- and low-level fields for causing electromigration and electroporation. In this application, a device being "configured" to produce an electromagnetic field in vivo means that (i) the portion of the device that comes in contact with body tissue or fluid is made of biocompatible materials, (ii) the electrodes are capable of carrying the current required for electroporation and/or electromigration of living cells in vivo in an electrolyte which may include the tissue being treated, interstitial fluid, injected material at the treatment site, material applied to the target tissue, and combinations of the foregoing, and (iii) the material between the electrodes on each support member, which may be the same material as the support member, should have a sufficient dielectric constant so that it does not break down as a result of nearby electrodes being of opposite polarity during electrical treatment. Moreover, it will be apparent to those skilled in the art that where an electrode or system is configured to perform both electromigration and electroporation, such an electrode or system may be used to perform either or both functions.

In the foregoing description, certain terms have been used for brevity, clarity, and understanding, but no unnecessary limitations are to be implied therefrom beyond the requirements of the prior art, because such words are used for description purposes herein and are intended to be broadly construed. Moreover, the embodiments of the apparatus illustrated and described herein are by way of example, and the scope of the invention is not limited to the exact details of construction.

Having now described the invention, the construction, the operation and use of preferred embodiment thereof, and the advantageous new and useful results obtained thereby, the new and useful constructions, and reasonable mechanical equivalents thereof obvious to those skilled in the art, are set forth in the appended claims.

## Claims

1. A device for manipulating a molecule (M) *in vivo* relating to a target tissue (T) comprising:
a support (11) and at least first and second members (12) affixed to and extending away from the support, the first member having at least one discrete electrode and the second member having at least two discrete electrodes, each electrode being in circuit communication with a respective portion of a source of electrical energy (50), the electrodes being disposed in axially spaced-apart relation along the member;
the discrete electrodes being configured to establish a first electromagnetic field *in vivo* between selected electrodes sufficient to cause an electromigration of said molecule relative to a target tissue and/or a second electromagnetic field sufficient to cause transient permeability of a cell membrane within the target tissue.

2. The device recited in Claim 1, wherein the second field is higher than the first field.

3. The device recited in Claim 1, wherein the member further has an insulating material axially interposed between the electrodes.

4. The device recited in Claim 1, wherein the member comprises: an elongated core electrode (13) comprising a conductive material; a nonconductive insulator sleeve (16) positioned in surrounding relation to a portion of the core electrode; and an outer electrode (14) positioned in surrounding relation to a portion of the sleeve, a bottom of the sleeve protruding therefrom.

5. The device recited in Claim 4, wherein the sleeve has a bottom portion adapted to protrude beneath a bottom of the outer electrode.

6. The device recited in Claim 4, wherein the outer electrode comprises a fist outer electrode and the sleeve comprises a first sleeve, and further comprising:
a second insulator sleeve (17) positioned in surrounding relation to a portion of the first outer electrode, a bottom portion of the first outer electrode protruding therefrom; and
a second outer electrode (15) positioned in surrounding relation to a portion of the second sleeve.

7. The device recited in Claim 6, wherein the first sleeve has a bottom portion positioned to protrude beneath a bottom of the first outer electrode and the second sleeve has a bottom portion adapted to protrude beneath a bottom of the second outer electrode.

8. The device recited in Claim 1, wherein the at least one member comprises a plurality of members disposed about the support in spaced relation from each other and configured to surround a periphery of at least a portion of the target tissue.

9. The device recited in Claim 1, wherein the at least one member comprises a pair of members disposed in spaced-apart and adapted to proved at least one pair of opposite-polarity voltages approximately simultaneously on at least one electrode on each member.

10. The device recited in Claim 1, further comprising means for selectively activating a selected plurality of electrodes in a predetermined pattern.

11. The device recited in Claim 1, wherein the member has a portal therein for distributing a molecule therethrough, the portal adjacent at least one of the electrodes.

12. The device recited in Claim 1, wherein the member comprises an elongated nonconductive post (18) and each electrode comprises a band wrapped at least partially circumferentially about the post, each electrode in spaced relation from an adjacent electrode.

13. The device recited in Claim 12, wherein the post comprises a plurality of posts affixed in speed-apart relation to the support for surrounding at least a portion of a target tissue.

14. The device recited in Claim 13, further comprising means for activating a pair of electrodes on different posts for providing an electromagnetic pulse across at least a portion of the target tissue.

15. The device recited in Claim 14, wherein the activating means comprises means for activating successive pairs of electrodes in a preselected pattern.

16. The device recited in Claim 1, further comprising means for activating each electrode in a preselected pattern for optimizing entry of the molecule into the target tissue.

## Patentansprüche

1. Vorrichtung zum Manipulieren eines Moleküls (M) *in vivo* in Bezug auf ein Zielgewebe (T), wobei die Vorrichtung Folgendes umfasst:
einen Träger (11) und wenigstens ein erstes und ein zweites Element (12), die an dem Träger befestigt sind und von diesem weg verlaufen, wobei das erste Element wenigstens eine diskrete Elektrode und das zweite Element wenigstens zwei diskrete Elektroden haben, wobei jede Elektrode in Schaltkreisverbindung mit einem jeweiligen Teil einer Quelle (50) von elektrischer Energie ist, wobei die Elektroden in axial beabstandeter Beziehung entlang des Elementes angeordnet sind;
wobei die diskreten Elektroden so konfiguriert sind, dass sie ein erstes elektromagnetisches Feld *in vivo* zwischen gewählten Elektroden, das ausreicht, um eine Elektromigration des genannten Moleküls relativ zu einem Zielgewebe zu erzeugen, und/oder ein zweites elektromagnetisches Feld erzeugen, das ausreicht, um eine transiente Permeabilität einer Zellmembran in dem Zielgewebe zu bewirken.

2. Vorrichtung nach Anspruch 1, wobei das zweite Feld größer ist als das erste Feld.

3. Vorrichtung nach Anspruch 1, wobei das Element ferner ein axial zwischen den Elektroden befindliches Isoliermaterial aufweist.

4. Vorrichtung nach Anspruch 1, wobei das Element Folgendes umfasst: eine längliche Kernelektrode (13), die ein leitendes Material umfasst; eine nichtleitende Isolierhülse (16), die sich in Umgebungsbeziehung zu einem Abschnitt der Kernelektrode befindet; und eine Außenelektrode (14), die sich in Umgebungsbeziehung zu einem Abschnitt der Hülse befindet, wobei ein Boden der Hülse davon vorsteht.

5. Vorrichtung nach Anspruch 4, wobei die Hülse einen unteren Abschnitt hat, der so gestaltet ist, dass er unter einem Boden der Außenelektrode vorsteht.

6. Vorrichtung nach Anspruch 4, wobei die Außenelektrode eine erste Außenelektrode und die Hülse eine erste Hülse umfasst, und die ferner Folgendes umfasst:
eine zweite Isolatorhülse (17), die sich in Umgebungsbeziehung zu einem Abschnitt der ersten Außenelektrode befindet, wobei ein unterer Abschnitt der ersten Außenelektrode davon vorsteht; und
eine zweite Außenelektrode (15), die sich in Umgebungsbeziehung zu einem Abschnitt der zweiten Hülse befindet.

7. Vorrichtung nach Anspruch 6, wobei die erste Hülse einen unteren Abschnitt aufweist, der so positioniert ist, dass er unter einem Boden der ersten Außenelektrode vorsteht, und wobei die zweite Hülse einen unteren Abschnitt hat, der so gestaltet ist, dass er unter einem Boden der zweiten Außenelektrode vorsteht.

8. Vorrichtung nach Anspruch 1, wobei das wenigstens eine Element eine Mehrzahl von Elementen umfasst, die um den Träger in beabstandeter Beziehung voneinander angeordnet und so konfiguriert sind, dass sie eine Peripherie von wenigstens einem Teil des Zielgewebes umgeben.

9. Vorrichtung nach Anspruch 1, wobei das wenigstens eine Element ein Paar Elemente umfasst, die in beabstandeter Beziehung angeordnet und so gestaltet sind, dass wenigstens ein Paar Spannungen von entgegengesetzter Polarität etwa gleichzeitig an wenigstens eine Elektrode an jedem Element angelegt werden.

10. Vorrichtung nach Anspruch 1, ferner umfassend Mittel zum selektiven Aktivieren einer gewählten Mehrzahl von Elektroden in einem vorbestimmten Muster.

11. Vorrichtung nach Anspruch 1, wobei in dem Element ein Portal vorgesehen ist, um ein Molekül dadurch zu verteilen, wobei sich das Portal neben wenigstens einer der Elektroden befindet.

12. Vorrichtung nach Anspruch 1, wobei das Element einen länglichen nichtleitenden Pfosten (18) umfasst und jede Elektrode ein Band umfasst, das wenigstens teilweise umfangsmäßig um den Pfosten gewickelt ist, wobei jede Elektrode eine beabstandete Beziehung zu einer benachbarten Elektrode hat.

13. Vorrichtung nach Anspruch 12, wobei der Pfosten eine Mehrzahl von Pfosten umfasst, die in beabstandeter Beziehung zu dem Träger befestigt sind, um wenigstens einen Teil eines Zielgewebes zu umgeben.

14. Vorrichtung nach Anspruch 13, ferner umfassend Mittel zum Aktivieren eines Paares von Elektroden an verschiedenen Pfosten zum Anlegen eines elektromagnetischen Impulses über wenigstens einen Teil des Zielgewebes.

15. Vorrichtung nach Anspruch 14, wobei das Aktivierungsmittel Mittel zum Aktivieren von aufeinander folgenden Paaren von Elektroden in einem vorgewählten Muster umfasst.

16. Vorrichtung nach Anspruch 1, ferner umfassend Mittel zum Aktivieren jeder Elektrode in einem vorgewählten Muster zum Optimieren des Eintretens des Moleküls in das Zielgewebe.

## Revendications

1. Dispositif pour manipuler une molécule (M) *in vivo,* ayant trait à un tissu cible (T), comprenant :
un élément porteur (11) et au moins un premier et second éléments (12) fixés à l'élément porteur et s'étendant en s'écartant de celui-ci, le premier élément ayant au moins une électrode discrète et le second élément ayant au moins deux électrodes discrètes, chaque électrode étant en communication de circuit avec une partie respective d'une source d'énergie électrique (50),
les électrodes étant disposées en une relation axialement écartée le long de l'élément ;
les électrodes discrètes étant configurées de sorte à établir un premier champ électromagnétique *in vivo* entre des électrodes sélectionnées, champ suffisant pour causer une électromigration de ladite molécule relativement à un tissu cible et/ou un second champ électromagnétique suffisant pour provoquer une perméabilité transitoire d'une membrane cellulaire à l'intérieur du tissu cible.

2. Le dispositif selon la revendication 1, dans lequel le second champ est plus fort que le premier champ.

3. Le dispositif selon la revendication 1, dans lequel l'élément est de plus pourvu d'un matériau isolant qui est interposé axialement entre les électrodes.

4. Le dispositif selon la revendication 1, dans lequel l'élément comprend : une électrode intérieure allongée (13) qui comprend un matériau conducteur ; une gaine isolante non conductrice (16) qui entoure une partie de l'électrode intérieure ; et une électrode extérieure (14) qui entoure une partie de la gaine, un fond de la gaine faisant saillie hors de celle-ci.

5. Le dispositif selon la revendication 4, dans lequel la gaine à une partie inférieure adaptée pour faire saillie au-dessous d'un fond de l'électrode extérieure.

6. Le dispositif selon la revendication 4, dans lequel l'électrode extérieure comprend une première électrode extérieure et la gaine comprend une première gaine, et qui comprend de plus :
une seconde gaine isolante (17) qui entoure une partie de la première électrode extérieure, une partie inférieure de la première électrode extérieure faisant saillie hors de celle-ci ; et
une seconde électrode extérieure (15) qui entoure une partie de la seconde gaine.

7. Le dispositif selon la revendication 6, dans lequel la première gaine a une partie inférieure placée de sorte à faire saillie au-dessous d'un fond de la première électrode extérieure et la seconde gaine a une partie inférieure adaptée pour faire saillie au-dessous d'un fond de la seconde électrode extérieure.

8. Le dispositif selon la revendication 1, dans lequel au moins un élément comprend une pluralité d'éléments disposés autour du support, écartés les uns des autres et configurés pour entourer une périphérie d'au moins une partie du tissu cible.

9. Le dispositif selon la revendication 1, dans lequel au moins un élément comprend une paire d'éléments disposés en une relation écartée et adaptés pour fournir au moins une paire de tensions à polarité opposée, de façon approximativement simultanée, sur au moins une électrode sur chaque élément.

10. Le dispositif selon la revendication 1, dans lequel qui comprend de plus des moyens pour activer sélectivement une pluralité sélectionnée d'électrodes selon une séquence prédéterminée.

11. Le dispositif selon la revendication 1, dans lequel l'élément est pourvu d'une ouverture pour distribuer une molécule à travers l'ouverture, l'ouverture étant adjacente à au moins une des électrodes.

12. Le dispositif selon la revendication 1, dans lequel l'élément comprend un support non conducteur allongé (18) et chaque électrode comprend une bande enroulée au moins partiellement et circonférentiellement autour de ce support, chaque électrode étant en relation espacée relativement à une électrode adjacente.

13. Le dispositif selon la revendication 12, dans lequel le support comprend une pluralité de supports fixés en une relation espacée à l'élément porteur d'électrodes pour entourer au moins une partie d'un tissu cible.

14. Le dispositif selon la revendication 13, qui comprend de plus des moyens pour activer une paire d'électrodes sur des supports différents afin de créer une impulsion électromagnétique aux bornes d'au moins une partie du tissu cible.

15. Le dispositif selon la revendication 14, dans lequel les moyens d'activation comprennent des moyens pour activer des paires successives d'électrodes selon une séquence présélectionnée.

16. Le dispositif selon la revendication 1, qui comprend de plus des moyens pour activer chaque électrode selon une séquence présélectionnée pour optimiser la pénétration de la molécule dans le tissu cible.
